# EUROPEAN PATENT SPECIFICATION

(11) **EP 2 953 540 B1**
(45) Date of publication and mention of the grant of the patent: **07.12.2016**
(21) Application number: 14703857.4
(22) Date of filing: 11.02.2014
(51) Int. Cl.: A61B 5/145, A61B 10/00, G01N 33/487, G01N 35/00, A61B 5/157, A61B 5/15

(54) **HANDHELD MEDICAL INSTRUMENT AND SYSTEM FOR ANALYZING A BODY FLUID**
TRAGBARES MEDIZINISCHES INSTRUMENT FÜR DIE ANALYSE VON KÖRPERFLUID
INSTRUMENT MÉDICAL PORTATIF POUR ANALYSE DE LIQUIDE CORPOREL

(30) Priority: 11.02.2013 EP 13154813
(43) Date of publication of application: 16.12.2015
(73) Proprietor: Roche Diabetes Care GmbH, 68305 Mannheim (DE); F.Hoffmann-La Roche AG, 4070 Basel (CH)
(72) Inventor: CHEN, Ming-Wen, Taipei City 106 (TW)
(74) Representative: Pfiz, Thomas
(86) International application number: PCT/EP2014/052662
(87) International publication number: WO 2014/122324

(56) References cited:
- EP-A1- 1 739 432
- EP-A1- 2 461 160
- WO-A1-01/23885
- WO-A1-2011/064178
- DE-A1- 4 427 363

## Description

The invention concerns a handheld medical instrument for analyzing a body fluid, in particular for blood glucose tests, comprising a housing having a cassette compartment construed for receiving an exchangeable tape cassette and a transport mechanism comprising a hand-operated manual drive which can be operated by a user for rotating a tape spool of the tape cassette, such that a test tape of the tape cassette is wound forwards onto the tape spool and functional elements stored thereon on the test tape are provided for successive use. The invention further concerns a system including such an instrument and a disposable tape cassette, and a method of operation.

Similar motorized devices are used in practice as blood glucose meters for the self-diagnosis of diabetics. A plurality of test fields is provided on a spoolable test tape in the tape cassette. The reactive test fields are examined photometrically after the application of a small amount of blood sample in order to determine the glucose content as exactly and reliably as possible. Such tape cassettes are intended to be inserted as a disposable part into a compact hand-held device housing in order to allow the necessary analytical steps to be carried out automatically and rapidly. For a simplified system design, it has already been proposed in EP-A 1 702 565 to use a tape cassette which can be hand-operated by means of a lever arranged externally of a device housing.

WO01/23885 A1 discloses a test device for test of an analyte concentration in a fluid, the device including a mechanism for indexing a reel comprising a ratchet wheel. However, the ratchet wheel does not interact with any other structure to have an impact on this movement for a targeted stop.

DE 44 27 363 A1 concerns a disposable chemical sensor including a sensor disk with circumferential notches, which act together with a spring catch independently of a transport mechanism formed by a turning knob.

On this basis the object of the invention is to further improve the known test instruments, systems and methods of operation and to achieve a reliable function in a simplified cost-efficient design.

The combination of features stated in claim 1 is proposed to achieve this object. Advantageous embodiments and further developments of the invention are derived from the dependent claims.

The invention is based on the idea of using an automatic device for securing proper tape positions. Accordingly it is proposed according to the invention a stop device acting on the transport mechanism and designed to stop the test tape in a functional position of a functional element. The stop device is constructed as an instrument unit to interrupt the tape transport if needed. Thereby, the handling is kept simple for the user, who can concentrate his attention to the drive operation. Moreover, it is possible to assume exact functional positions thereby guaranteeing reliable measurements even under conditions of varying functional positions and tape transport distances during consecutive tests. Then, the user need not dispense with improved convenience provided by a large number of tests storable on a tape and easiness of disposal of used material on a waste tape spool. Manufacture of the instrument can be simplified as compared to automatically driven devices, and the risk of a motor stall is avoided. Overall, the power consumption of the device can be minimized and high power internal supply is superfluous.

In a preferred embodiment the stop device has a position detecting unit preferably comprising a contact-free scanning sensor adapted for detecting a functional position of a functional element on the test tape. Thus, a targeted stop can be achieved even in case of tape slippage or distance tolerances of the functional elements on teh tape. Nonconctact scanning also allows for exact positioning without need for complicated tape design or mechanical engagement. The position detecting unit may directly or indirectly detect the functional element in a given position. In the latter case, the stop device may have an optical sensor adapted to detect position marks on the test tape and to provide a trigger signal to stop the test tape.

Another particularly advantageous embodiment provides that the stop device has a locking unit which can be actuated for mechanical blocking of the transport mechanism. This provides a secure stop with simple measures.

In order to further allow a precise actuation, it is advantageous when the stop device has a solenoid-operated locking actuator and a return spring to provide a locking force against rotation of the tape spool when the actuator is released.

It is also advantageous when the stop device has a control member which is linked to the manual drive to prevent a blocking of the transport mechanism in an initial transport phase. Thereby, it is also possible to avoid excessive power consumption.

In order to prevent excessive forces on the test tape, it is advantageous when the transport mechanism can be switched to an idle state when reaching a functional position by means of the stop device.

For a further reduction of the constructional effort it is also conceivable that the stop device has a position indication unit for providing a visual, acoustical or haptic perceptible instruction to the user to stop actuation of the manual drive, thus acting on the transport mechanism in a very simple way via the user.

A further improvement in safe and reliable hand-operation can be achieved when the manual drive comprises a swivel-mounted or linear guided push bar which can be operated by the user and a pinion which engages teeth on the push bar and converts the motion of the push bar into a rotational motion to rotate the tape spool.

A further improvement in this regard is based on a transport mechanism having a freewheel assembly that allows a rotary motion transferred to the tape spool in only one direction while preventing motion in the opposite direction.

For a reliable torque transmission it is advantageous when the transport mechanism has a gear-wheel which can be rotated by the manual drive and which can be blocked by the stop device, and wherein the gear-wheel is directly coupled to the tape spool by a connecting journal when the tape cassette is inserted in the cassette compartment.

In order to avoid excessive loads, it is advantageous when the transport mechanism has a speed indication unit for providing a visual, acoustical or haptic perceptible signal to the user to indicate the rotational speed of the tape spool.

It is also conceivable that the transport mechanism includes an electric energy generator which can be operated by the manual drive and an electric motor supplied with energy from the generator preferably via a super capacitor as a storage means.

For further automation of the positioning it can be advantageous when the transport mechanism includes a storage means for storing mechanical energy, preferably a spring which can be loaded by the manual drive in order to automatically rotate the tape spool in a transport cycle.

In order to secure reliable measurements the test tape can have consecutive tape sections each including a functional element formed as a test field for application of body fluid and at least one additional functional element, specifically a calibration field. Generally, the term "functional element" as used herein denotes a distinctive element or field on the test tape which can be positioned by tape transport to assume a functional position in which an instrument operation, specifically a measurement mode is supported or triggered.

The invention also concerns a medical system for analyzing a body fluid, in particular for blood glucose tests, comprising a tape cassette including a test tape and a handheld instrument, the instrument comprising:
a housing having a cassette compartment construed for receiving the tape cassette,
a transport mechanism comprising a manual drive which can be actuated by a user in order to rotate a tape spool of the tape cassette, such that the test tape is wound forwards and functional elements stored thereon are provided for successive use,
a stop device acting on the transport mechanism and designed to stop the test tape in a functional position of a functional element provided on the test tape.
With respect to advantageous effects of such a system, reference is made to the aforegoing statements.

Procedurally, it is proposed a method for operating a handheld medical instrument for analyzing a body fluid comprising the following steps
- inserting an exchangeable tape cassette comprising a test tape into a cassette compartment provided in a housing of the instrument,
- actuating a manual drive of a transport mechanism in order to rotate a tape spool of the tape cassette, such that the test tape is wound forwards and functional elements stored thereon are provided for successive use,
- stopping the test tape in a functional position of a functional element by means of an automatic stop device acting on the transport mechanism.
Again, with respect to advantageous effects of such a method, reference is made to the aforegoing explanations.

The invention is further elucidated in the following on the basis of an embodiment example shown schematically in the drawings, where
- Fig. 1: is a top view of a cassette-type blood glucose meter with test tape transport mechanism including a hand-operated drive;
- Fig. 2: is a block diagram of the blood glucose meter;
- Fig. 3: is a schematic view of a stop device acting on the tape transport mechanism;
- Fig. 4: is a more detailed top view of the tape transport mechanism in cooperation with the stop device in an initial position;
- Fig. 5: is an expanded view of Fig. 4;
- Fig. 6 to 8: show sequential positions following the initial position shown in Fig. 4;
- Fig. 9: is a top view of a section of the test tape.

The drawings show a medical instrument configured as a portable blood glucose meter 10 for self-monitoring of blood glucose and comprising a housing 12 with a cassette compartment 14 defining a space for inserting a disposable tape cassette 16 and a transport mechanism 18 for rotating a tape spool 20 of the tape cassette 16, such that an analytical test tape 22 of the cassette can be wound forwards and functional elements 24 stored thereon can be sequentially provided for use, specifically for application and investigation of a blood sample.

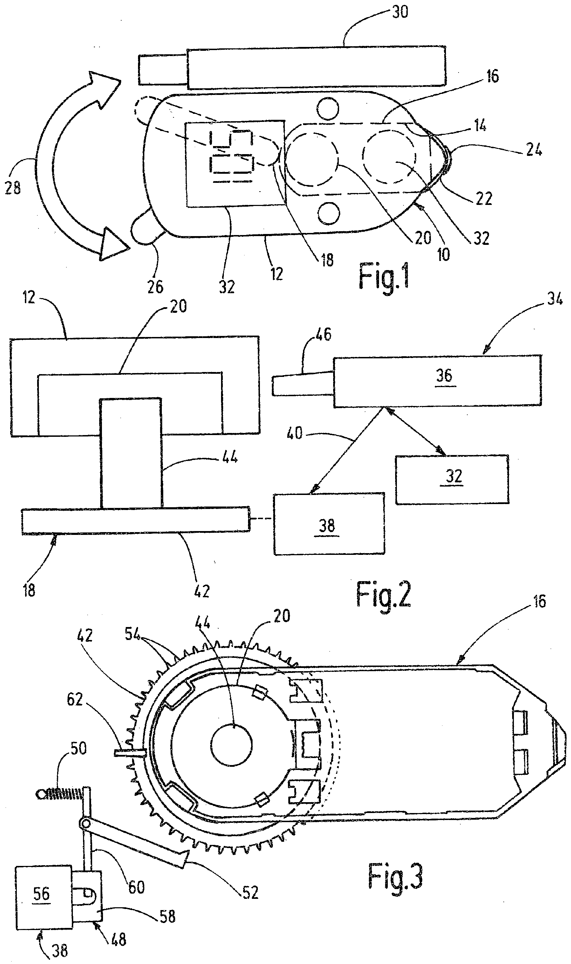

As illustrated in Fig. 1, the transport mechanism 18 comprises a manual drive 26 which can be operated by a user in bidirectional movement as depicted by double arrow 28. The system including the tape cassette 16 may also be equipped with a lancing aid 30, allowing the user to draw a fresh sample of whole blood on-the-spot and to apply the sample at an application tip of the cassette 16. The on-board photometric detection of the analyte (glucose) is known per se in the state of the art and needs not be explained in further detail. The test result may be rapidly provided on a display 32. The tape spool 20 is intended for taking or winding-up the used section of the test tape 22, whereas a storage 32 shields unused tape preferably on an unwinding spool. Thus, the flexible test tape 22 is only transported via rotation of the spool 20, so that sprocket holes can be avoided in the thin tape foil.

Fig. 2 further exemplifies that the instrument 10 comprises a stop device 34 that acts on the transport mechanism 18 to achieve a targeted stop of the test tape 22 in a functional position, for example when a functional element 24 in the form of a reactive test field is positioned on the tip of the cassette 16. The stop device 34 comprises a position detecting unit 36 scanning the test tape 22 during its transport and a locking unit 38 which can be actuated by a trigger signal 40 of the position detecting unit 36 for mechanical blocking of the transport mechanism 18. For this purpose, the transport mechanism 18 comprises a rotating unit 42, e.g. a gear-wheel which is directly coupled to the tape spool 20 by a connecting journal 44 when the tape cassette 20 is inserted in the housing 12.

In a practical embodiment, the position detecting unit 36 has an optical sensor 46 to detect position marks on the test tape 22, as explained in more detail further below. Furthermore, the position detecting unit 36 may be connected to the display 32 to give a positional feedback to the user. In a very simple arrangement, such feedback may be intended to cause the user to stop operating the manual drive 26, thereby acting on the transport mechanism 18 via the user.

Fig. 3 illustrates the function of the locking unit 38 of the stop device 34. The gear-wheel 42 directly drives the tape spool 20 of the tape cassette 16. The locking unit 38 comprises a solenoid-operated actuator 48 and a return spring 50 to provide a locking force via a latch 52 that engages the teeth 54 of the gear-wheel 42. In the unlocked state, the solenoid coil 56 is energized and the solenoid core 58 is retrieved (to the left in Fig. 3). At the same time, the return spring 50 is biased or extended, and the latch 52 is out of engagement, thereby allowing the gear-wheel 42 to rotate. Upon receiving a trigger signal 40, the coil 56 is de-energized and the core 58 is drawn out (to the right in Fig. 3) under the return force of the contracting return spring 50. As the lever 60 linking the actuator 48 and the spring 50 is tilted, the latch 52 locks against the teeth 54 and prevents further motion of the gear-wheel 42.

It may be desirable to let the user feel the proper rotational speed when operating the manual drive. In a very simple embodiment, gear-wheel 42 as shown in Fig. 3 may form a dial for a direct manual rotation. Then, a mark spring 62 on the gear-wheel 42 can provide a haptic perceptible signal or feedback to the user to indicate the present revolutions or speed of rotation.

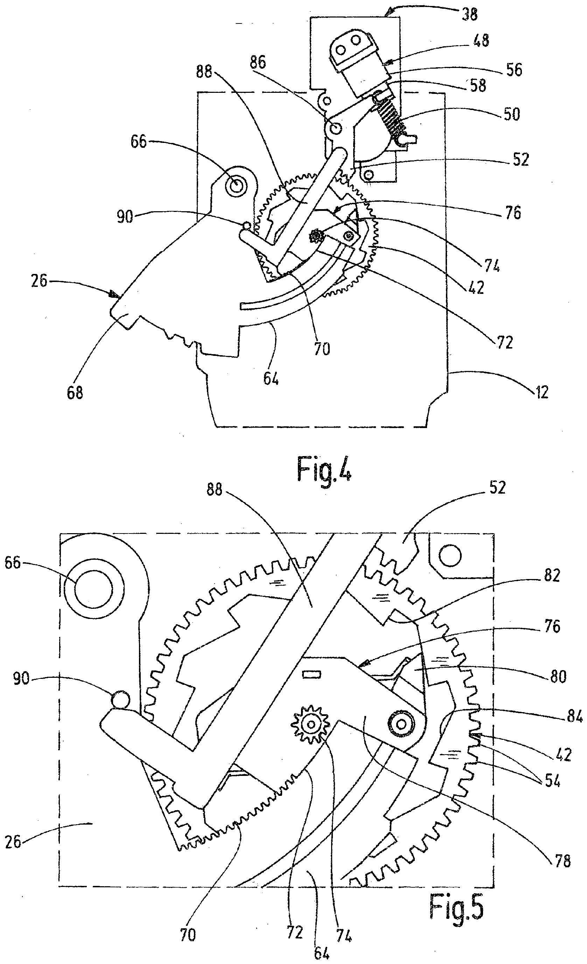

Fig. 4 shows a manual drive 26 comprising a push-bar 64 which is swivel-mounted at a swivel-bearing 66 and can be hand-operated by means of a handle 68. The push-bar 64 has an arched toothed segment 70 and an override segment 72 without teeth towards its free end.

As can be also seen from Fig. 5, a rotatable pinion 74 is mounted in the movement path of the push-bar 64, so that it can be rotated by the toothed segment 70. The pinion 74 is connected to a freewheel assembly 76 in a rotatably fixed manner. The freewheel assembly 76 has a polygonal freewheel body 78 which is provided at opposed ends with two swivel-mounted pawls 80. The pawls 80 are springloaded against the inner asymmetrically saw-toothed contour of the gear-wheel 42, which is connectable to the tape spool 20 as explained above.

In the course of manual operation, when the handle 68 is pushed inwards to the housing 12, the pinion 74 eventually engages the toothed segment 70 and converts the motion of the push-bar 64 into a rotation of the body 78 in anticlockwise direction. The pawls 80 will then catch against the first steeply sloped inner edge 80 of the gear-wheel 42, thereby locking it for cooperative movement with the freewheel body 78. When the push-bar 64 is pivoted in the opposite (outward) direction, however, the freewheel body 78 rotates in clockwise direction and the pawls 80 can easily slide up and over the gently sloped chamfers 84 of the gear-wheel 42, thereby allowing a free rotation of the assembly 76 without actuation of the gear-wheel 42.

Turning back now to Fig. 4 the locking unit 38 of the stop device 34 is shown to have a similar arrangement as already explained above. The solenoid-operated actuator 48 is connected at its core 58 to return spring 50, which is expanded in the initial state. The latch 52 is formed as a two-armed lever which is swivel-mounted at swivel-bearing 86. In the initial state, the latch 52 does not engage the teeth 54 of the gear-wheel 42.

In order to secure this initial state independently of the current feed state of the solenoid coil 56, the latch 52 is linked to the push-bar 68 of the manual drive 26 by means of an L-shaped control arm 88. This arm 88 is supported at its shorter bracket by means of a support pin 90 projecting at the side of the push-bar 64. Thereby, the return force of the return spring 50 can be hold solely mechanical, without current consumption in the coil 56.

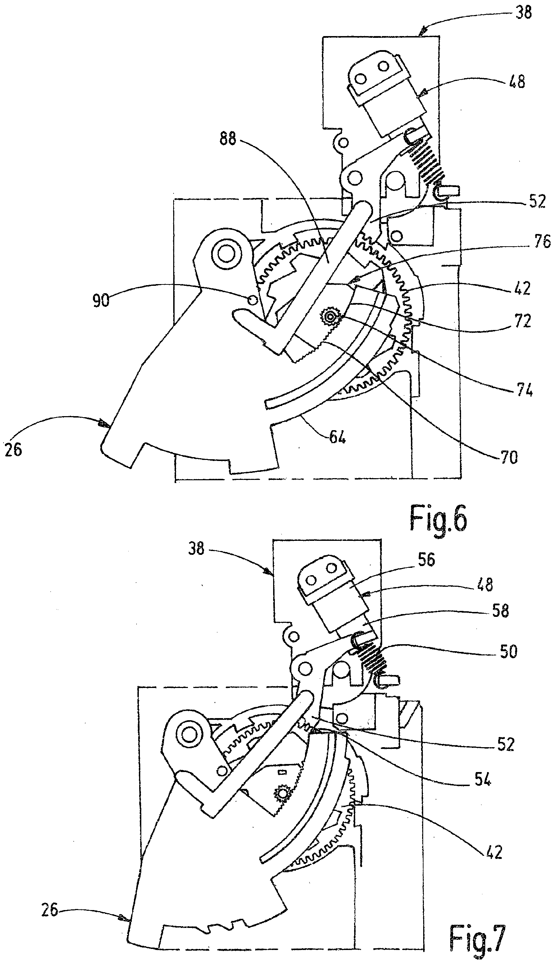

As apparent from Fig. 6, in the beginning phase of the manual operation of the push-bar 64, the tooth-free override segment 72 moves past the pinion 74 without engagement, however, allowing the control arm 88 to come clear from the support pin 90. In this phase, the solenoid-operated actuator 48 should be already energized to prevent unwanted blocking of the gear-wheel 42. The current feed may be achieved by means of a switch (not shown) actuated by the push-bar 64. Upon further movement of the push-bar 64, the gear-wheel 42 is rotated through the toothed segment 70, and the test tape 22 is advanced by means of the tape spool 20.

Fig. 7 shows the blocking state in which the locking unit 38 has received a trigger signal, and the solenoid-operated actuator 48 is switched-off. Then, the return spring 50 contracts, whereby the core 58 is drawn out of the coil 56, and the latch 52 is pivoted into the blocking state where the teeth 54 of the gear-wheel 42 are engaged. Thereby, the test tape 22 is stopped in a functional position.

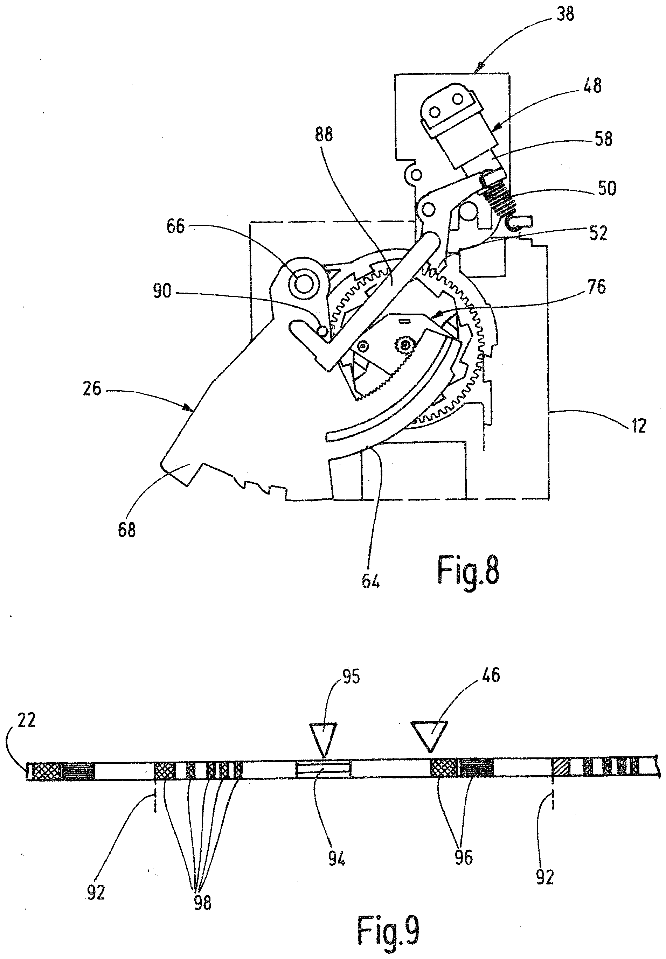

Fig. 8 shows a return state in which the push-bar 64 has swung back (outwards of the housing 12) under the influence of a torsion spring which may be arranged in the bearing 66. This motion occurs automatically when the user releases the handle 68, however only to an angle distance where the support pin 90 catches the control arm 90 in the center of the short bracket. Then, in order to reach the initial state shown in Fig. 4, the user has to actively swivel out the handle 68, whereby the return spring 50 is extended, the core 58 is moved back and the latch 52 is secured through the control arm 88 supported at pin 90.

Fig. 9 illustrates a test tape 22 that has consecutive sections 92 (for example, a total of fifty sections 92) that are each used for a single measurement. Body fluid is supplied to an analytical test field 94 and investigated by means of an optical measurement unit 95 of the instrument 10 in a defined functional (measurement) position. In addition, calibration fields 96 are provided in each section 92 which can be used for a calibration of the measurement unit 95 when properly aligned. For a targeted stop of the test tape 22, each section 92 contains position marks 98 which can be detected by the optical sensor 46 of the position detection unit 36. Apparently, the tape transport distances vary in order to approach the different functional positions of the functional elements 24, i.e. the test field 94 and the calibration fields 96. Moreover the diameter of the tape wrap on the tape spool 20 increases with increasing use of tests. These difficulties can be overcome by the cooperative action of the stop device 34 and the manual drive 26.

## Claims

1. Handheld medical instrument for analyzing a body fluid, in particular for blood glucose tests, comprising
a) a housing (12) having a cassette compartment (14) construed for receiving an exchangeable tape cassette (16),
b) a transport mechanism (18) comprising a manual drive which can be actuated by a user in order to rotate a tape spool (20) of the tape cassette (16), wherein a test tape (22) of the tape cassette (16) is wound forwards and functional elements (24) stored thereon are provided for successive use,
**characterized by**
c) a stop device (34) acting on the transport mechanism (18) and designed to stop the test tape (22) in a functional position of a functional element (24).

2. Medical instrument according to claim 1, wherein the stop device (34) has a position detecting unit (36) preferably comprising a contact-free scanning sensor (46) adapted for detecting a functional position of a functional element (24) provided on the test tape (22).

3. Medical instrument according to claim 1 or 2, wherein the stop device (34) has an optical sensor (46) adapted to detect position marks (98) on the test tape (22) and to provide a trigger signal to stop the test tape (22).

4. Medical instrument according to one of the claims 1 to 3, wherein the stop device (34) has a locking unit (38) which can be actuated for mechanical blocking of the transport mechanism (18).

5. Medical instrument according to one of the claims 1 to 4, wherein the stop device (34) has a solenoid-operated locking actuator (48) and a return spring (50) to provide a locking force against rotation of the tape spool (20) when the locking actuator (48) is released.

6. Medical instrument according to one of the claims 1 to 5, wherein the transport mechanism (18) can be switched to an idle state when reaching a functional position by means of the stop device (34).

7. Medical instrument according to one of the claims 1 to 6, wherein the stop device (34) has a position indication unit (32) for providing a visual, acoustical or haptic perceptible instruction to the user to stop actuation of the manual drive.

8. Medical instrument according to one of the claims 1 to 7, wherein the manual drive comprises a swivel-mounted or linear guided push bar (64) which can be operated by the user and a pinion (74) which engages teeth (70) on the push bar (64) and converts the motion of the push bar (64) into a rotational motion to rotate the tape spool (20).

9. Medical instrument according to one of the claims 1 to 8, wherein the transport mechanism (18) has a freewheel assembly (76) that allows a rotary motion transferred to the tape spool (20) in only one direction while preventing motion in the opposite direction.

10. Medical instrument according to one of the claims 1 to 9, wherein the transport mechanism (18) has a gear-wheel (42) which can be rotated by the manual drive and which can be blocked by the stop device (34), and wherein the gear-wheel (42) is directly coupled to the tape spool (20) by a connecting journal (44) when the tape cassette (16) is inserted in the cassette compartment (14).

11. Medical instrument according to one of the claims 1 to 10, wherein the transport mechanism (18) includes an electric energy generator which can be operated by the manual drive and an electric motor supplied with energy from the generator preferably via a super capacitor as a storage means.

12. Medical instrument according to one of the claims 1 to 11, wherein the transport mechanism (18) includes a storage means for storing mechanical energy, preferably a spring which can be loaded by the manual drive in order to automatically rotate the tape spool (20) in a transport cycle.

13. Medical instrument according to one of the claims 1 to 12, wherein the test tape (22) has consecutive tape sections (92) each including a functional element formed as a test field (94) for application of body fluid and at least one additional functional element (96), specifically a calibration field, and wherein different tape transport distances are required to reach a respective functional position of the functional elements (24) in each tape section (92).

14. Medical system for analyzing a body fluid, in particular for blood glucose tests, comprising a tape cassette (16) including a test tape (22) and a handheld instrument according to one of claims 1 to 13, the instrument comprising:
a) a housing (12) having a cassette compartment (14) construed for receiving the tape cassette (16),
b) a transport mechanism (18) comprising a manual drive which can be actuated by a user in order to rotate a tape spool (20) of the tape cassette (16), such that the test tape (22) is wound forwards and functional elements (24) stored thereon are provided for successive use,
**characterized by**
c) a stop device (34) acting on the transport mechanism (18) and designed to stop the test tape (22) in a functional position of a functional element (24) provided on the test tape (22).

15. Method for operating a handheld medical instrument for analyzing a body fluid, in particular the instrument according to one of claims 1 to 13, comprising the following steps
a) inserting an exchangeable tape cassette (16) comprising a test tape (22) into a cassette compartment (14) provided in a housing (12) of the instrument,
b) actuating a manual drive of a transport mechanism (18) in order to rotate a tape spool (20) of the tape cassette (16), such that the test tape (22) is wound forwards and functional elements (24) stored thereon are provided for successive use,
**characterized by**
c) stopping the test tape (22) in a functional position of a functional element (24) by means of an automatic stop device (34) acting on the transport mechanism (18).

## Patentansprüche

1. Tragbares medizinisches Gerät zum Analysieren einer Körperflüssigkeit, insbesondere für Glukosetests, mit
a) einem Gehäuse (12), das ein zum Aufnehmen einer austauschbaren Bandkassette (16) ausgelegtes Kassettenfach (14) hat,
b) einem Transportmechanismus (18) umfassend einen manuellen Antrieb, der von einem Benutzer zum Rotieren einer Bandspule (20) der Bandkassette (16) betätigbar ist, wobei ein Testband (22) der Bandkassette (16) vorgespult wird und darauf gelagerte Funktionselemente (24) zum aufeinanderfolgenden Gebrauch bereitgestellt werden,
**gekennzeichnet durch**
c) eine auf den Transportmechanismus (18) einwirkende Stoppvorrichtung (34), die dazu ausgebildet ist, das Testband (22) in einer Funktionsposition eines Funktionselements (24) zu stoppen.

2. Medizinisches Gerät nach Anspruch 1, bei dem die Stoppvorrichtung (34) eine vorzugsweise einen berührungsfreien Abtastsensor (46) umfassende Positionserfassungseinheit (36) aufweist, welche zum Erfassen einer Funktionsposition eines auf dem Testband (22) bereitgestellten Funktionselements (24) ausgebildet ist.

3. Medizinisches Gerät nach Anspruch 1 oder 2, bei dem die Stoppvorrichtung (34) einen optischen Sensor (46) aufweist, der dazu ausgebildet ist, Positionsmarken (98) auf dem Testband (22) zu erfassen und ein Triggersignal zum Stoppen des Testbands (22) zu liefern.

4. Medizinisches Gerät nach einem der Ansprüche 1 bis 3, bei dem die Stoppvorrichtung (34) eine Sperreinheit (38) aufweist, die zum mechanischen Sperren des Transportmechanismus (18) betätigt werden kann.

5. Medizinisches Gerät nach einem der Ansprüche 1 bis 4, bei dem die Stoppvorrichtung (34) einen mittels magnetbetätigten Sperraktuator (48) und eine Rückstellfeder (50) aufweist, um beim Auslösen des Sperraktuators (48) eine Sperrkraft gegen Rotation der Bandspule (20) auszuüben.

6. Medizinisches Gerät nach einem der Ansprüche 1 bis 5, bei dem der Transportmechanismus (18) beim Erreichen einer Funktionsposition mittels der Stoppvorrichtung (34) in einen Leerlauf geschaltet werden kann.

7. Medizinisches Gerät nach einem der Ansprüche 1 bis 6, bei dem die Stoppvorrichtung (34) eine Positionsanzeigeeinheit (32) zur Bereitstellung einer visuellen, akustischen oder haptisch wahrnehmbaren Anweisung an den Benutzer zum Stoppen der Betätigung des manuellen Antriebs aufweist.

8. Medizinisches Gerät nach einem der Ansprüche 1 bis 7, bei dem der manuelle Antrieb eine schwenkbar gelagerte oder linear geführte, durch den Benutzer betätigbare Druckstange (64) und ein Antriebsritzel (74) umfasst, das mit Zähnen (70) an der Druckstange (64) kämmt und die Bewegung der Druckstange (64) in eine Rotationsbewegung zum Rotieren der Bandspule (20) überträgt.

9. Medizinisches Gerät nach einem der Ansprüche 1 bis 8, bei dem der Transportmechanismus (18) eine Freilaufeinrichtung (76) aufweist, welche eine auf die Bandspule (20) übertragene Rotationsbewegung nur in einer Richtung zulässt und Bewegung in Gegenrichtung verhindert.

10. Medizinisches Gerät nach einem der Ansprüche 1 bis 9, bei dem der Transportmechanismus (18) ein durch den manuellen Antrieb drehbares und durch die Stoppvorrichtung (34) sperrbares Zahnrad (42) umfasst, wobei das Zahnrad (42) bei eingelegter Bandkassette (16) in dem Kassettenfach (14) durch einen Verbindungszapfen (44) direkt mit der Bandspule (20) gekoppelt ist.

11. Medizinisches Gerät nach einem der Ansprüche 1 bis 10, bei dem der Transportmechanismus (18) einen mit dem manuellen Antrieb betreibbaren elektrischen Energiegenerator und einen vorzugsweise über einen Superkondensator als Speichermittel mit Energie aus dem Generator versorgten elektrischen Motor umfasst.

12. Medizinisches Gerät nach einem der Ansprüche 1 bis 11, bei dem der Transportmechanismus (18) ein Speichermittel zur Speicherung von mechanischer Energie, vorzugweise eine durch den manuellen Antrieb spannbare Feder umfasst, um die Bandspule (20) in einem Transportzyklus automatisch zu drehen.

13. Medizinisches Gerät nach einem der Ansprüche 1 bis 12, bei dem das Testband (22) aufeinanderfolgende Testbandabschnitte (92) aufweist, von denen jeder ein als Testfeld (94) zur Applikation von Körperflüssigkeit ausgebildetes Funktionselement und zumindest ein weiteres Funktionselement (96), insbesondere ein Kalibrierfeld umfasst, wobei verschiedene Transportstrecken erforderlich sind, um eine jeweilige Funktionsposition der Funktionselemente (24) in jedem Bandabschnitt (92) zu erreichen.

14. Medizinisches System zum Analysieren einer Körperflüssigkeit, insbesondere für Blutzuckertests, umfassend eine ein Testband (22) enthaltende Bandkassette (16) und ein tragbares Gerät nach einem der Ansprüche1 bis 13, wobei das Gerät umfasst:
a) ein Gehäuse (12) mit einem zur Aufnahme der Bandkassette (16) ausgelegten Kassettenfach (14),
b) einen Transportmechanismus (18) umfassend einen durch einen Benutzer betätigbaren manuellen Antrieb zum Drehen einer Bandspule (20) der Bandkassette (16), so dass das Testband (22) vorgespult wird und darauf gelagerte Funktionselemente (24) zum aufeinanderfolgenden Gebrauch bereitgestellt werden,
**gekennzeichnet durch**
c) eine auf den Transportmechanismus (18) einwirkende Stoppvorrichtung (34), die dazu ausgebildet ist, das Testband (22) in einer Funktionsposition eines auf dem Testband (22) bereitgestellten Funktionselements (24) zu stoppen.

15. Verfahren zum Betrieb eines tragbaren medizinischen Geräts zur Analyse einer Körperflüssigkeit, insbesondere des Geräts nach einem der Ansprüche 1 bis 13, mit den folgenden Schritten
a) Einsetzen einer ein Testband (22) enthaltenden austauschbaren Bandkassette (16) in ein in einem Gehäuse (12) des Geräts vorgesehenes Kassettenfach (14),
b) Betätigen eines manuellen Antriebs des Transportmechanismus (18) zum Rotieren einer Bandspule (20) der Bandkassette (16), so dass das Testband (22) vorgespult wird und darauf gelagerte Funktionselemente (24) zum aufeinanderfolgenden Gebrauch bereitgestellt werden,
**gekennzeichnet durch**
c) Stoppen des Testbands (22) in einer Funktionsposition eines Funktionselements (24) mittels einer auf den Transportmechanismus (18) einwirkenden automatischen Stoppvorrichtung (34).

## Revendications

1. Instrument médical portatif permettant d'analyser un fluide corporel, en particulier destiné à des tests de glycémie, comprenant
a) une enveloppe (12) présentant un compartiment à cassette (14) conçu pour accueillir une cassette à bande (16) pouvant être échangée,
b) un mécanisme de transport (18) comprenant un entraînement manuel qui peut être actionné par un utilisateur afin de faire tourner une bobine de bande (20) de la cassette à bande (16), dans lequel une bande de test (22) de la cassette à bande (16) est enroulée vers l'avant et des éléments fonctionnels (24) stockés sur celle-ci sont fournis en vue d'utilisations successives,
**caractérisé par**
c) un dispositif d'arrêt (34) agissant sur le mécanisme de transport (18) et conçu pour arrêter la bande de test (22) dans une position fonctionnelle d'un élément fonctionnel (24).

2. Instrument médical selon la revendication 1, dans lequel le dispositif d'arrêt (34) présente une unité de détection de position (36) comprenant de manière préférée un capteur à balayage sans contact (46) conçu pour détecter une position fonctionnelle d'un élément fonctionnel (24) fourni sur la bande de test (22).

3. Instrument médical selon la revendication 1 ou 2, dans lequel le dispositif d'arrêt (34) présente un capteur optique (46) conçu pour détecter des repères de position (98) sur la bande de test (22) et pour fournir un signal de déclenchement afin d'arrêter la bande de test (22).

4. Instrument médical selon l'une quelconque des revendications 1 à 3, dans lequel le dispositif d'arrêt (34) présente une unité de verrouillage (38) qui peut être actionnée en vue d'un blocage mécanique du mécanisme de transport (18).

5. Instrument médical selon l'une quelconque des revendications 1 à 4, dans lequel le dispositif d'arrêt (34) présente un actionneur de verrouillage (48) commandé par électro-aimant et un ressort de rappel (50) afin de fournir une force de verrouillage à l'encontre d'une rotation de la bobine de bande (20) lorsque l'actionneur de verrouillage (48) est libéré.

6. Instrument médical selon l'une quelconque des revendications 1 à 5, dans lequel le mécanisme de transport (18) peut être commuté vers un état de repos lorsqu'il atteint une position fonctionnelle au moyen du dispositif d'arrêt (34).

7. Instrument médical selon l'une quelconque des revendications 1 à 6, dans lequel le dispositif d'arrêt (34) présente une unité d'indication de position (32) permettant de fournir à l'utilisateur une instruction, perceptible de manière visuelle, acoustique ou haptique, l'invitant à arrêter l'actionnement de l'entraînement manuel.

8. Instrument médical selon l'une quelconque des revendications 1 à 7, dans lequel l'entraînement manuel comprend une barre-poussoir (64) montée sur pivot ou guidée de manière linéaire que l'utilisateur peut faire fonctionner et un pignon (74) qui vient en prise avec des dents (70) situées sur la barre-poussoir (64) et convertit le mouvement de la barre-poussoir (64) en un mouvement rotatif afin de faire tourner la bobine de bande (20).

9. Instrument médical selon l'une quelconque des revendications 1 à 8, dans lequel le mécanisme de transport (18) présente un ensemble roue libre (76) qui permet un transfert de mouvement rotatif vers la bobine de bande (20) dans seulement une direction tout en empêchant un mouvement dans la direction opposée.

10. Instrument médical selon l'une quelconque des revendications 1 à 9, dans lequel le mécanisme de transport (18) présente une roue d'engrenage (42) que l'entraînement manuel peut faire tourner et qui peut être bloquée grâce au dispositif d'arrêt (34), et dans lequel la roue d'engrenage (42) est couplée directement à la bobine de bande (20) grâce à un tourillon de raccordement (44) lorsque la cassette à bande (16) est insérée dans le compartiment à cassette (14).

11. Instrument médical selon l'une quelconque des revendications 1 à 10, dans lequel le mécanisme de transport (18) comprend un générateur d'énergie électrique que l'entraînement manuel peut faire fonctionner et un moteur électrique alimenté en énergie par ledit générateur, de manière préférée via un supercondensateur faisant office de moyen de stockage.

12. Instrument médical selon l'une quelconque des revendications 1 à 11, dans lequel le mécanisme de transport (18) comprend un moyen de stockage permettant de stocker de l'énergie mécanique, de manière préférée un ressort qui peut être mis en tension grâce à l'entraînement manuel afin de faire tourner de manière automatique la bobine de bande (20) dans un cycle de transport.

13. Instrument médical selon l'une quelconque des revendications 1 à 12, dans lequel la bande de test (22) présente des sections de bande (92) consécutives comprenant respectivement un élément fonctionnel sous forme d'un champ de test (94) destiné à une application de fluide corporel et au moins un élément fonctionnel (96) supplémentaire, de manière spécifique un champ d'étalonnage, et dans lequel différentes distances de transport de bande sont nécessaires pour atteindre une position fonctionnelle respective des éléments fonctionnels (24) dans chaque section de bande (92).

14. Système médical permettant une analyse d'un fluide corporel, en particulier destiné à des tests de glycémie, comprenant une cassette à bande (16) comprenant une bande de test (22) et un instrument portatif selon l'une quelconque des revendications 1 à 13, l'instrument comprenant :
a) une enveloppe (12) présentant un compartiment à cassette (14) conçu pour accueillir la cassette à bande (16),
b) un mécanisme de transport (18) comprenant un entraînement manuel qui peut être actionné par un utilisateur afin de faire tourner une bobine de bande (20) de la cassette à bande (16), de telle manière que la bande de test (22) est enroulée vers l'avant et des éléments fonctionnels (24) stockés sur celle-ci sont fournis en vue d'utilisations successives,
**caractérisé par**
c) un dispositif d'arrêt (34) agissant sur le mécanisme de transport (18) et conçu pour arrêter la bande de test (22) dans une position fonctionnelle d'un élément fonctionnel (24) fourni sur la bande de test (22).

15. Procédé permettant de faire fonctionner un instrument médical portatif en vue d'une analyse d'un fluide corporel, en particulier instrument selon l'une quelconque des revendications 1 à 13, comprenant les étapes suivantes consistant à
a) insérer une cassette à bande (16) échangeable comprenant une bande de test (22) dans un compartiment à cassette (14) fourni dans une enveloppe (12) de l'instrument,
b) actionner un entraînement manuel d'un mécanisme de transport (18) afin de faire tourner une bobine de bande (20) de la cassette à bande (16), de telle manière que la bande de test (22) est enroulée vers l'avant et des éléments fonctionnels (24) stockés sur celle-ci sont fournis en vue d'utilisations successives,
**caractérisé par** l'étape consistant à
c) arrêter la bande de test (22) dans une position fonctionnelle d'un élément fonctionnel (24) au moyen d'un dispositif d'arrêt automatique (34) agissant sur le mécanisme de transport (18).
